# EUROPEAN PATENT APPLICATION

(11) **EP 2 730 920 A1**
(43) Date of publication of application: **14.05.2014**
(21) Application number: 12811126.7
(22) Date of filing: 11.01.2012
(51) Int. Cl.: G01N 30/88, G01N 30/60

(54) **METHOD FOR MEASURING HEMOGLOBINS**

(30) Priority: 08.07.2011 JP 2011152148
(71) Applicant: Sekisui Medical Co., Ltd., Tokyo 103-0027 (JP)
(72) Inventor: OISHI Kazuyuki, Ryugasaki-shi Ibaraki 301-0852 (JP)
(74) Representative: Hart-Davis, Jason
(86) International application number: PCT/JP2012/050309
(87) International publication number: WO 2013/008479

(57) **Abstract**

The present invention aims to provide a method of measuring hemoglobins which can measure hemoglobins in a short time at high accuracy. The present invention also aims to provide a method of measuring hemoglobin A1c, a method of simultaneously measuring hemoglobin A1c and hemoglobin F, a method of simultaneously measuring hemoglobin A1c and hemoglobin A2, and a method of simultaneously measuring hemoglobin A1c and abnormal hemoglobins, each utilizing the above-mentioned method of measuring hemoglobins by liquid chromatography. The present invention relates to a method of measuring hemoglobins by liquid chromatography. A column used in the method is filled with, as a column-packing material, cation-exchangeable particles including cross-linked polymer particles having a cation-exchange-group-containing polymer bonded to the surface of the cross-linked polymer particles, and the column shows a pressure value of 9.8 × 10³ Pa or higher and 29.4 × 10⁵ Pa or lower when an eluent for measurement is delivered at 1.0 mL/min.

## Description

### TECHNICAL FIELD

The present invention relates to a method of measuring hemoglobins by liquid chromatography. The present invention also relates to a method of measuring hemoglobin A1c, a method of simultaneously measuring hemoglobin A1c and hemoglobin F, a method of simultaneously measuring hemoglobin A1c and hemoglobin A2, and a method of simultaneously measuring hemoglobin A1c and abnormal hemoglobins, each utilizing the above-mentioned method of measuring hemoglobins by liquid chromatography.

### BACKGROUND ART

Methods of measuring hemoglobins by liquid chromatography can measure hemoglobins in a short time at higher accuracy than other measurement methods. Such methods utilizing liquid chromatography are thus used for controlling the hemoglobin A1c levels in diabetics.

In conventional methods of measuring hemoglobins by liquid chromatography, an eluent is delivered at a flow rate faster than a predetermined rate in order to achieve short-time measurement or fine packing material particles are used in order to maintain high accuracy. Such operations cause a high pressure value in the measurement system.

In the technique disclosed in Patent Literature 1, for example, an eluent delivered at a rate of 1.0 mL/min gives a pressure value in the measurement system of approximately 5 MPa. The pressure value generated in the measurement system of common measurement of hemoglobins is equal to or higher than that disclosed in Patent Literature 1. Thus, conventional methods unavoidably cause measurement at high-pressure conditions in order to achieve measurement in a short time or at high accuracy, and such methods therefore use high-pressure-resistant devices.

However, such measurement at high-pressure conditions gives a higher load of pressure on the channel and this load causes slight liquid leakage from joint portions of the channel or pulsating currents, resulting in a deformed chromatogram. Finally, the measurement accuracy is deteriorated in some cases. Further, in comparison with low-pressure conditions, such high-pressure conditions have the pressure value strongly influenced by, for example, the presence of non-specific adsorption to the channels including columns and filters, switching of eluents in the case of using multiple eluents, and a change in environmental temperature, and thus may deteriorate the measurement accuracy. These phenomena may also occur with the use of a high-pressure resistant device.

### CITATION LIST

### - Patent Literature

Patent Literature 1: JP H05-005730 A

### SUMMARY OF INVENTION

### - Technical Problem

The present invention aims to provide a method of measuring hemoglobins which enables to measure hemoglobins in a short time at high accuracy. The present invention also aims to provide a method of measuring hemoglobin A1c, a method of simultaneously measuring hemoglobin A1c and hemoglobin F, a method of simultaneously measuring hemoglobin A1c and hemoglobin A2, and a method of simultaneously measuring hemoglobin A1c and abnormal hemoglobins, each utilizing the above-mentioned method of measuring hemoglobins by liquid chromatography which enables to measure hemoglobins in a short time at high accuracy.

### - Solution to Problem

The present inventor has found that, in a method of measuring hemoglobins using a column filled with a column-packing material having a predetermined structure, the use of a column showing a pressure value within a predetermined range under predetermined conditions enables to improve the repeatability of the measurement. This pressure value range is different from the pressure value ranges of columns used in conventional methods of measuring hemoglobins. Thus, the present inventor also has found that the pressure value of the column used in the present invention is significantly lower than the pressure values of conventional columns.

The present invention relates to a method of measuring hemoglobins by liquid chromatography, in which a column is used, the column being filled with, as a column-packing material, cation-exchangeable particles including cross-linked polymer particles having a cation-exchange-group-containing polymer bonded to the surface of the cross-linked polymer particles, and the column showing a pressure value of 9.8 × 10³ Pa or higher and 29.4 × 10⁵ Pa or lower when an eluent for the measurement is delivered at 1.0 mL/min.

The present invention will be described in detail below.

The column-packing material used in the method of measuring hemoglobins of the present invention is cation-exchangeable particles including cross-linked polymer particles having a cation-exchange-group-containing polymer bonded to the surface of the cross-linked polymer particles.

The cross-linked polymer particles are preferably particles formed by polymerizing organic cross-linkable monomers or a monomer mixture mainly containing organic cross-linkable monomers. More preferable are particles formed by polymerizing cross-linkable acrylic monomers or a monomer mixture mainly containing cross-linkable acrylic monomers. The cross-linked polymer particles are preferably particles that have substantially no ion exchangeability.

The term "acrylic" herein means the presence of an acryl group or a methacryl group. The term "(meth)acrylic acid" herein means "acrylic acid or methacrylic acid", and the term "(meth)acrylate" herein means "acrylate or methacrylate".

Examples of the cross-linkable acrylic monomer include polyethylene glycol di(meth)acrylates, polypropylene glycol di(meth)acrylates, alkylene glycol di(meth)acrylates, hydroxyalkyl di(meth)acrylates, and alkylolalkane (meth)acrylates having at least two (meth)acryl groups in the molecule.

Examples of the polyethylene glycol di(meth)acrylates include ethylene glycol di(meth)acrylate, diethylene glycol di(meth)acrylate, triethylene glycol di(meth)acrylate, tetraethylene glycol di(meth)acrylate, and polyethylene glycol di(meth)acrylate.

Examples of the polypropylene glycol di(meth)acrylates include propylene glycol di(meth)acrylate, dipropylene glycol di(meth)acrylate, tripropylene glycol di(meth)acrylate, tetrapropylene glycol di(meth)acrylate, and polypropylene glycol di(meth)acrylate.

Examples of the alkylene glycol di(meth)acrylates include polytetramethylene glycol di(meth)acrylate, poly(propylene glycol-tetramethylene glycol)-di(meth)acrylate, polyethylene glycol polypropylene glycol polyethylene glycol-di(meth)acrylate, 1,3-butylene glycol di(meth)acrylate, 1,4-butylene glycol di(meth)acrylate, 1,6-hexaglycol di(meth)acrylate, 1,9-nonanediol di(meth)acrylate, and neopentyl glycol di(meth)acrylate.

Examples of the hydroxyalkyl di(meth)acrylates include 2-hydroxy-1,3-di(meth)acryloxypropane, 2-hydroxy-1-(meth)acryloxy-3-(meth)acryloxypropane, 2-hydroxy-3-(meth)acryloyloxypropyl (meth)acrylate, glycerol di(meth)acrylate, glycerol acrylate methacrylate, urethane di(meth)acrylate, isocyanuric acid di(meth)acrylate, isocyanuric acid tri(meth)acrylate, 1,10-di(meth)acryloxy-4,7-dioxadecane-2,9-diol, 1,10-di(meth)acryloxy-5-methyl-4,7-dioxadecane-2,9-diol, and 1,11-di(meth)acryloxy-4,8-dioxaundecane-2,6,10-triol.

Examples of the alkylolalkane (meth)acrylates having at least two (meth)acryl groups in the molecule include trimethylolpropane tri(meth)acrylate, tetramethylolpropane tri(meth)acrylate, ditrimethylolpropane tetra(meth)acrylate, tetramethylolmethane tri(meth)acrylate, tetramethylolmethane tetra(meth)acrylate, and trimethylolethane tri(meth)acrylate.

Each of these cross-linkable acrylic monomers may be used alone, or two or more of these may be used in combination. These cross-linkable acrylic monomers are preferably in the form of having no ion-exchange group.

The monomers constituting the cross-linked polymer particles may further include a non-cross-linkable monomer.

The non-cross-linkable monomer is preferably a non-cross-linkable acrylic monomer.

The non-cross-linkable acrylic monomer is preferably a hydrophilic non-cross-linkable acrylic monomer, and examples thereof include alkyl (meth)acrylates and non-cross-linkable acrylic monomers having a hydroxy group.

Examples of the alkyl (meth)acrylates include methyl (meth)acrylate, ethyl (meth)acrylate, propyl (meth)acrylate, butyl (meth)acrylate, and 2-ethylhexyl (meth)acrylate.

Examples of the non-cross-linkable acrylic monomers having a hydroxy group include polyethylene glycol mono(meth)acrylates, polypropylene glycol mono(meth)acrylates, alkylene glycol mono(meth)acrylates, and other (meth)acrylates having a hydroxy group.

Examples of the polyethylene glycol mono(meth)acrylates include ethylene glycol mono(meth)acrylate, diethylene glycol mono(meth)acrylate, triethylene glycol mono(meth)acrylate, tetraethylene glycol mono(meth)acrylate, polyethylene glycol mono(meth)acrylate, methoxytri(poly)ethylene glycol mono(meth)acrylate, and methoxy polyethylene glycol mono(meth)acrylate.

Examples of the polypropylene glycol mono(meth)acrylates include propylene glycol mono(meth)acrylate, dipropylene glycol mono(meth)acrylate, tripropylene glycol mono(meth)acrylate, tetrapropylene glycol mono(meth)acrylate, and polypropylene glycol mono(meth)acrylate.

Examples of the alkylene glycol mono(meth)acrylates include poly(ethylene glycol-propylene glycol)mono(meth)acrylate, poly(ethylene glycol-tetramethylene glycol)mono(meth)acrylate, poly(propylene glycol-tetramethylene glycol)mono(meth)acrylate, and octoxypolyethylene glycol polypropylene glycol-mono(meth)acrylate.

Examples of other (meth)acrylates having a hydroxy group include glycerin mono(meth)acrylate, 2-hydroxyethyl (meth)acrylate, 2-hydroxypropyl (meth)acrylate, 2,3-dihydroxylethyl (meth)acrylate, and 2,3-dihydroxylpropyl (meth)acrylate.

Each of these non-cross-linkable acrylic monomers may be used alone, or two or more of these may be used in combination. These non-cross-linkable acrylic monomers are preferably in the form of having no ion-exchange group.

In the case of using a non-cross-linkable monomer as one of the monomers constituting the cross-linked polymer particles, the upper limit of the amount of the non-cross-linkable monomer in the mixture of a cross-linkable monomer and a non-cross-linkable monomer is 30% by weight. If the amount of the non-cross-linkable monomer exceeds 30% by weight, the pressure resistance and the swelling resistance of the column-packing material to be obtained may be poor, failing to accurately measure hemoglobins. The upper limit of the amount of the non-cross-linkable monomer is more preferably 20% by weight, and still more preferably 10% by weight.

The cross-linkable monomer or the mixture of a cross-linkable monomer and a non-cross-linkable monomer (hereinafter, they are also collectively referred to as the "cross-linkable monomer species") may be polymerized by any of conventionally known methods in the presence of a polymerization initiator such as emulsion polymerization, soap-free polymerization, dispersion polymerization, suspension polymerization, and seed polymerization. Preferable are dispersion polymerization, suspension polymerization, and seed polymerization.

In the case of suspension polymerization, for example, a polymerization initiator is dissolved in a cross-linkable monomer species and the resulting mixture is dispersed in an appropriate dispersion medium. Then, the dispersion is warmed under stirring in an inert gas (e.g. nitrogen gas) atmosphere as appropriate. This provides cross-linked polymer particles suitable as a column-packing material.

The polymerization initiator used for polymerizing a cross-linkable monomer species may be a known water-soluble or oil-soluble radical polymerization initiator. Examples thereof include persulfates such as potassium persulfate, sodium persulfate, and ammonium persulfate; organic peroxides such as cumene hydroperoxide, benzoyl peroxide, lauroyl peroxide, octanoyl peroxide, o-chlorobenzoyl peroxide, acetyl peroxide, t-butyl hydroperoxide, t-butyl peroxyacetate, t-butyl peroxyisobutyrate, 3,5,5-trimethyl hexanoyl peroxide, t-butyl peroxy-2-ethylhexanoate, and di-t-butyl peroxide; and azo compounds such as 2,2-azobisisobutyronitrile, 2,2-azobis(2,4-dimethylvaleronitrile), 4,4-azobis(4-cyanopentanoic acid), 2,2-azobis(2-methylbutyronitrile), 2,2-azobis(2,4-dimethylvaleronitrile), and azobiscyclohexanecarbonitrile.

With respect to the amount of the polymerization initiator, the lower limit is preferably 0.05 parts by weight, whereas the upper limit is preferably 5 parts by weight, for 100 parts by weight of the cross-linkable monomer species. If the amount of the polymerization initiator is less than 0.05 parts by weight, unreacted monomers remain to have adverse effects on measurement of hemoglobins. Long-time polymerization for polymerizing unreacted monomers may cause aggregation of monomers during the polymerization, failing to form particles. If the amount of the polymerization initiator exceeds 5 parts by weight, the polymerization reaction may drastically proceed to generate an aggregate.

Known additives may be added upon polymerization of the cross-linkable monomer species. Examples of such additives include porosity-control agents for forming macropores in the cross-linked polymer particles, various kinds of chain-transfer agents for controlling polymerization reaction, and dispersants for stabilizing suspension particles.

The column-packing material used in the method of measuring hemoglobins of the present invention includes cation-exchangeable particles including cross-linked polymer particles having a cation-exchange-group-containing polymer bonded to the surface of the cross-linked polymer particles.

Examples of the cation exchange group include a carboxyl group, a phosphate group, and a sulfonic group. A sulfonic group is preferred. The cation-exchange-group-containing polymer may have various cation exchange groups.

The "cation exchange group" herein may have any accompanying structure. Thus, the "cation exchange group" herein includes all the functional groups terminated with a cation exchange group. For example, the term "carboxyl group" herein includes a carboxyl ethyl group, a carboxyl propyl group, and the like.

The cation-exchange-group-containing polymer is preferably a polymer obtained by polymerizing cation-exchange-group-containing monomers, or a polymer obtained by polymerizing monomers having a functional group that can be transformed into a cation exchange group and then transforming the functional group into a cation exchange group. Preferable is a polymer obtained by polymerizing cation-exchange-group-containing monomers.

The cation-exchange-group-containing monomer (hereinafter, also referred to as the cation exchangeable monomer) is a monomer having a polymerization-reactive functional group and a cation exchange group.

The cation exchangeable monomer is preferably a cation-exchange-group-containing acrylic monomer.

Examples of the acrylic monomer having a carboxyl group include (meth)acrylic acid derivatives such as (meth)acrylic acid, 2-(meth)acryloyloxyethyl succinate, and 2-(meth)acryloyloxyethyl phthalate.

Examples of the acrylic monomer having a phosphate group include (meth)acrylic acid derivatives such as ((meth)acryloyloxyethyl)acid phosphate, (2-(meth)acryloyloxyethyl)acid phosphate, and (3-(meth)acryloyloxypropyl)acid phosphate.

Examples of the acrylic monomer having a sulfonic group include (meth)acrylic acid derivatives such as 2-(meth)acrylamido-2-methylpropanesulfonic acid, 2-sulfoethyl (meth)acrylate, and 3-sulfopropyl (meth)acrylate.

Each of these cation exchangeable monomers may be used alone, or two or more of these may be used in combination.

In addition to these cation exchangeable monomers, a non-cross-linkable hydrophilic monomer may also be used. The non-cross-linkable hydrophilic monomer is preferably the above-mentioned non-cross-linkable acrylic monomer having a hydroxy group. Examples thereof include polyethylene glycol mono(meth)acrylates, polypropylene glycol mono(meth)acrylates, alkylene glycol mono(meth)acrylates, and other (meth)acrylates having a hydroxy group.

The cation-exchangeable particles having the aforementioned cation exchangeable monomer polymerized on the surface of the cross-linked polymer particles are preferably particles including the cation exchangeable monomer polymerized in the presence of cross-linked polymer particles and a polymerization initiator. More preferable are particles including the cation exchangeable monomer polymerized in the presence of cross-linked polymer particles containing a polymerization initiator therein.

The cross-linked polymer particles containing a polymerization initiator therein are preferably particles prepared as follows: first, the polymerization initiator is dissolved in an organic solvent that can swell cross-linked polymer particles and can dissolve the polymerization initiator; and then cross-linked polymer particles are immersed in this organic solvent.

Examples of the cation-exchangeable particles including a cation exchangeable monomer polymerized on the surface of the cross-linked polymer particles include particles prepared by introducing a polymerization initiator into the cross-linked polymer particles, dispersing the cross-linked polymer particles in an appropriate dispersion medium, adding a cation exchangeable monomer to the dispersion medium, and polymerizing the monomer.

The cation-exchangeable particles are still more preferably particles obtained by, in the polymerization reaction for preparing the cross-linked polymer particles, adding a cation exchangeable monomer to the reaction system before the polymerization reaction is completed, that is, before the polymerization initiator is completely consumed, and thereby continuing the polymerization reaction. Such particles are particles including the cation exchangeable monomer efficiently polymerized on the surface of the cross-linked polymer particles utilizing the polymerization initiator initially added for the polymerization reaction of the cross-linked polymer particles.

The cation-exchangeable particles are also preferably particles prepared by polymerizing a monomer having a functional group that can be transformed into a cation exchange group on the surface of the cross-linked polymer particles, and transforming the functional group into a cation exchange group.

The functional group that can be transformed into a cation exchange group (hereinafter, also referred to as the reactive group) is preferably a nonionic hydrophilic group. Examples of the nonionic hydrophilic group include a hydroxy group, a glycol group, an epoxy group, a glycidyl group, a primary amino group, a secondary amino group, a cyano group, and an aldehyde group. Preferable are a hydroxy group, an epoxy group, a glycidyl group, a primary amino group, and a secondary amino group, and more preferable are a hydroxy group, an epoxy group, and a glycidyl group.

The monomer having such a reactive group (hereinafter, also referred to as the reactive monomer) is preferably a (meth)acrylic acid ester.

Examples of the (meth)acrylic acid ester include polyethylene glycol mono(meth)acrylates, polypropylene glycol (meth)acrylates, alkylene glycol mono(meth)acrylates, epoxidized or hydroxylated (meth)acrylates, aminated (meth)acrylates, and aldehyde-modified or cyanized (meth)acrylates.

Examples of the polyethylene glycol mono(meth)acrylates include ethylene glycol mono(meth)acrylate, diethylene glycol mono(meth)acrylate, triethylene glycol mono(meth)acrylate, tetraethylene glycol mono(meth)acrylate, polyethylene glycol mono(meth)acrylate, methoxy tri(poly)ethylene glycol mono(meth)acrylate, and methoxy polyethylene glycol mono(meth)acrylate.

Examples of the polypropylene glycol (meth)acrylates include propylene glycol mono(meth)acrylate, dipropylene glycol mono(meth)acrylate, tripropylene glycol mono(meth)acrylate, tetrapropylene glycol mono(meth)acrylate, and polypropylene glycol mono(meth)acrylate.

Examples of the alkylene glycol mono(meth)acrylates include poly(ethylene glycol-propylene glycol)mono(meth)acrylate, poly(ethylene glycol-tetramethylene glycol)mono(meth)acrylate, poly(propylene glycol-tetramethylene glycol)mono(meth)acrylate, and octoxypolyethylene glycol polypropylene glycol-mono(meth)acrylate.

Examples of the epoxidized or hydroxylated (meth)acrylates include glycidyl (meth)acrylate, glycerin mono(meth)acrylate, 2-hydroxyethyl (meth)acrylate, 2-hydroxypropyl (meth)acrylate, 2,3-dihydroxylethyl (meth)acrylate, and 2,3-dihydroxylpropyl (meth)acrylate.

Examples of the aminated (meth)acrylates include 2-aminoethyl (meth)acrylate, 2,3-diaminoethyl (meth)acrylate, 2-aminopropyl (meth)acrylate, 2,3-diaminopropyl (meth)acrylate, and (meth)acrylamide.

The aldehyde-modified or cyanized (meth)acrylates include (meth)acrolein, cyano(meth)acrylate, and ethyl-2-acrylate.

Each of these reactive monomers may be used alone, or two or more of these may be used in combination.

The particles obtained by polymerizing the above reactive monomer on the surface of the cross-linked polymer particles are preferably particles obtained by polymerizing a reactive monomer in place of the cation exchangeable monomer. The above particles are particles obtained by polymerizing a reactive monomer in the presence of cross-linked polymer particles and a polymerization initiator. More preferable are particles obtained by polymerizing a reactive monomer in the presence of the cross-linked polymer particles containing a polymerization initiator therein.

The particles obtained by polymerizing a reactive monomer on the surface of the cross-linked polymer particles and transforming the reactive group thereon into a cation exchange group are preferably particles obtained by reacting the reactive group with a cation exchange group-containing compound (hereinafter, also referred to as the cation-exchangeable compound).

Examples of the particles on which the reactive group is reacted with a cationic compound include particles on which the reactive group is reacted with a cation-exchangeable compound by a known chemical reaction. Examples thereof include cation-exchangeable particles having a sulfonic group or a carboxyl group on the surface of the cross-linked polymer particles obtained by reacting the cross-linked polymer particles having a hydroxy group as a reactive group on the surface and a cation-exchangeable compound such as a halogenated ethanesulfonic acid (e.g. sodium bromoethanesulfonate) and a halogenated acetic acid (e.g. sodium chloroacetate) in an alkali hydroxide aqueous solution.

Examples thereof also include cation-exchangeable particles having a cation exchange group on the surface of the cross-linked polymer particles obtained by acetalizing cross-linked polymer particles having a hydroxy group as a reactive group on the surface with an aldehyde compound having a cation exchange group as the cation-exchangeable compound in the presence of an acid catalyst.

Examples thereof further include cation-exchangeable particles having a carboxyl group on the surface of the cross-linked polymer particles obtained by dehydration-esterifying the cross-linked polymer particles having a hydroxy group as a reactive group on the surface with a polyfunctional carboxylic compound (e.g. tricarballylic acid, butanetetracarboxylic acid) as a cation-exchangeable compound.

Examples thereof furthermore include cation-exchangeable particles having a sulfonic group on the surface of the cross-linked polymer particles obtained by reacting the cross-linked polymer particles having a hydroxy group on the surface as a reactive group with a cation-exchangeable compound (e.g. 1,3-propanesultone, 1,4-butanesultone) in an alkali hydroxide aqueous solution or an organic solvent.

The particles on which a reactive group is reacted with a cationic compound are the following particles (1), (2), or (3):
(1) cation-exchangeable particles having a sulfonic group or a carboxyl group on the surface of the cross-linked polymer particles obtained by reacting cross-linked polymer particles having an epoxy group or a glycidyl group as a reactive group on the surface with a cation-exchangeable compound such as sodium sulfate, taurine, or glycolic acid;
(2) cation-exchangeable particles having a sulfonic group on the surface of the cross-linked polymer particles obtained by heating cross-linked polymer particles having an epoxy group or a glycidyl group as a reactive group on the surface, boron trifluoride etherate, and sodium sulfite as a cation-exchangeable compound; or
(3) cation-exchangeable particles having a cation exchange group on the surface of the cross-linked polymer particles obtained by reacting cross-linked polymer particles having an amino group as a reactive group on the surface and an epoxy compound such as epichlorohydrin or triglycidyl ether in a solution of an alkali hydroxide in water or in an organic solvent to be epoxidized, and then performing the same treatment as in the case of an epoxy group or a glycidyl group.

With respect to the volume average particle size of the cation-exchangeable particles, the lower limit thereof is preferably 5 µm, whereas the upper limit thereof is preferably 30 µm. The cation-exchangeable particles with a volume average particle size of smaller than 5 µm may increase the pressure value when an eluent is delivered, making it difficult to provide a column having a pressure value within the range defined in the present invention. The cation-exchangeable particles with a volume average particle size of greater than 30 µm may increase the void in the column. This may cause a diffusion of a measurement sample and the peak is likely to be broader, resulting in a decrease in measurement accuracy. The lower limit of the volume average particle size of the cation-exchangeable particles is more preferably 6 µm, whereas the upper limit thereof is more preferably 25 µm.

With respect to the column filled with cation-exchangeable particles, the pressure value of the column increases as the volume average particle size of the cation-exchangeable particles is decreased, whereas the pressure value of the column decreases as the volume average particle size is increased. Thus, the pressure value of the column can be finely adjusted as follows: the pressure value can be decreased by increasing the volume average particle size of the cation-exchangeable particles, and the pressure value can be increased by decreasing the volume average particle size of the cation-exchangeable particles.

The column used in the method of measuring hemoglobins of the present invention is a column that is filled with the cation-exchangeable particles and has a pressure value of 9.8 × 10³ Pa or higher and 29.4 × 10⁵ Pa or lower when an eluent for the measurement is delivered at a rate of 1.0 mL/min.

In the following, the pressure value of a column when an eluent for the measurement is delivered at a rate of 1.0 mL/min is also referred to as the "column pressure value", and the pressure value ranged from 9.8 × 10³ Pa to 29.4 × 10⁵ Pa is also referred to as the "specified column pressure value". The term "pressure value" herein means a value shown by a manometer when the manometer is placed between a delivery pump and the column and an eluent for the measurement is delivered at the aforementioned rate.

A column with a column pressure value of lower than 9.8 × 10³ Pa fails to stabilize the pressure value in the measurement system, resulting in poor measurement accuracy. Further, the measurement may take a long time. A column with a column pressure value of higher than 29.4 × 10⁵ Pa leads to measurement under high pressure, resulting in poor measurement accuracy. The column to be used in the method of measuring hemoglobins of the present invention is preferably a column with a column pressure value of 4.9 × 10⁴ Pa or higher and 24.5 × 10⁵ Pa or lower.

In the method of measuring hemoglobins of the present invention, an empty column to be filled with the cation-exchangeable particles as a column-packing material may be an empty column made of a known material, such as metal, plastic, or glass. The empty column may have a known structure including a column body accommodating the column-packing material and an end-fitting including a frit.

With respect to the column body to be used in the method of measuring hemoglobins of the present invention, the lower limit of its length is preferably 3 mm, whereas the upper limit of its length is preferably 70 mm. The column body with a length of shorter than 3 mm may provide insufficient interaction between packing material and hemoglobins, thereby resulting in poor separation performance and poor measurement accuracy. The column body with a length of longer than 70 mm may cause a longer measurement time and may make it difficult to set the column pressure value to the specified column pressure value. The lower limit of the length of the column body is more preferably 5 mm, whereas the upper limit thereof is more preferably 50 mm.

The shorter the length of the column body becomes, the lower the column pressure value provided thereby would be. The longer the length of the column body becomes, the higher the column pressure value provided thereby would be. Thus, the column pressure value can be finely adjusted as follows: the pressure value can be decreased by shortening the column body, whereas the pressure value can be increased by lengthening the column body.

With respect to the column body to be used in the method of measuring hemoglobins of the present invention, the lower limit of the internal diameter thereof is preferably 1 mm, whereas the upper limit thereof is preferably 10 mm. The column body with an internal diameter of less than 1 mm may make it difficult to set the column pressure value to the specified column pressure value, resulting in poor measurement accuracy. The column with an internal diameter of greater than 10 mm may cause a sample to diffuse in the column, resulting in poor measurement accuracy. Further, the measurement time may be extended. The lower limit of the internal diameter of the column body is more preferably 2 mm, whereas the upper limit thereof is more preferably 4 mm.

The smaller the internal diameter of the column body becomes, the higher the column pressure value provided thereby would be. The greater the internal diameter of the column body becomes, the lower the column pressure value provided thereby would be. Thus, the column pressure value can be finely adjusted as follows: the column pressure value can be decreased by increasing the internal diameter of the column body, whereas the column pressure value can be increased by decreasing the internal diameter of the column body.

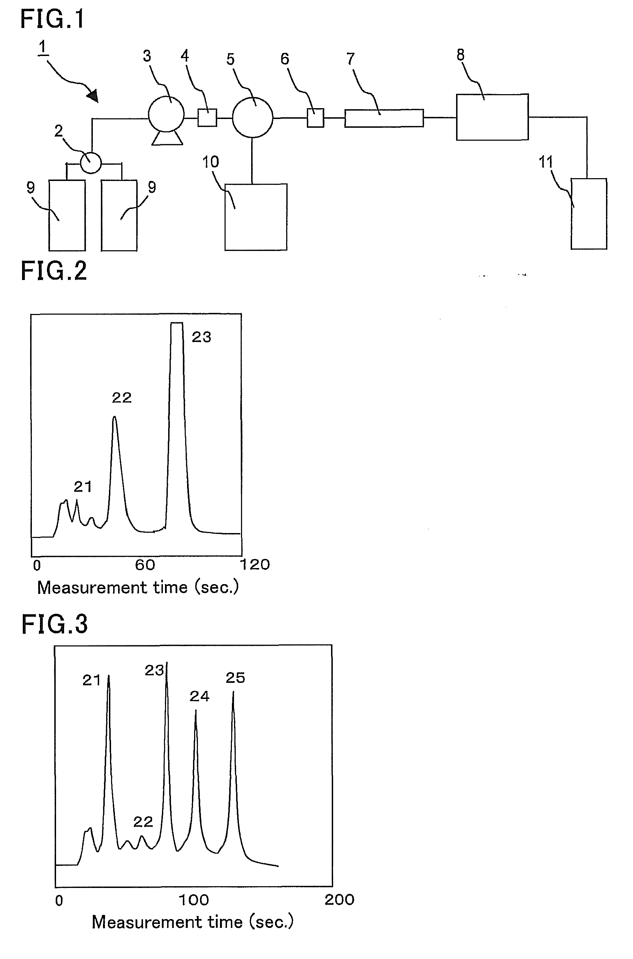

The method of measuring hemoglobins of the present invention may be performed using a known liquid chromatograph connected with the aforementioned column. The liquid chromatograph may be equipped with, for example, a mechanism for introducing a measurement sample, a pump for delivering an eluent, and a detector for detecting hemoglobins. Fig. 1 shows one exemplary configuration of a liquid chromatograph used in the method of measuring hemoglobins of the present invention.

The eluent to be used in the method of measuring hemoglobins of the present invention is preferably an organic solvent or a buffer containing a known salt compound, and more preferably a buffer.

Examples of the buffer include organic acids, inorganic acids, salts thereof, amino acids, and Good's buffer.

Examples of the organic acid include citric acid, succinic acid, tartaric acid, and malic acid.

Examples of the inorganic acid include hydrochloric acid, nitric acid, sulfuric acid, phosphoric acid, boric acid, and acetic acid.

Examples of the amino acids include glycine, taurine, and arginine.

Commonly used known components such as surfactants, polymers, low-molecular-weight hydrophilic compounds, and chaotropic ions may be appropriately added to the above buffers.

With respect to the buffer to be used in the measurement of hemoglobins, the lower limit of the salt concentration is preferably 10 mmol/L, whereas the upper limit thereof is preferably 1000 mmol/L. The buffer with a salt concentration of less than 10 mmol/L may lead to insufficient ion-exchange reaction, failing to separate hemoglobins. The buffer with a salt concentration of more than 1000 mmol/L may suffer precipitation of salts, and the salts may adversely affect the system.

For the measurement of hemoglobins, the pressure value in the measurement system is preferably 9.8 × 10³ Pa or higher and 19.6 × 10⁵ Pa or lower. In the following, the pressure value in the measurement system upon measurement is also referred to as the "pressure value in measurement", and the pressure value ranged from 9.8 × 10³ Pa to 19.6 × 10⁵ Pa is also referred to as the "specified pressure value in measurement". When the measurement is performed at a flow rate of 1.0 mL/min, the column pressure value corresponds to the pressure value in measurement.

If the pressure value in measurement is lower than 9.8 × 10³ Pa, the pressure value in measurement is less likely to be stabilized, resulting in poor measurement accuracy. Further, the measurement time may be extended. If the pressure value in measurement is higher than 19.6 × 10⁵ Pa, the measurement is performed under high pressure, resulting in poor measurement accuracy.

The pressure value in measurement can be adjusted by, for example, increasing or decreasing the rate of delivering an eluent.

The method of measuring hemoglobins of the present invention can measure various hemoglobins. For example, the method can measure healthy human hemoglobins: hemoglobin A0, hemoglobin A1c, hemoglobin F (fetal hemoglobin), and hemoglobin A2. In addition, the method can measure parts of hemoglobins called abnormal hemoglobins. Examples of the abnormal hemoglobins include hemoglobin S, hemoglobin C, hemoglobin D, and hemoglobin E.

The phrase "can measure the hemoglobins" herein means that the method can show the ratio of the peak area of each hemoglobin to the sum of all the peak areas or the sum of parts of the peak areas in the chromatogram, and thereby to give each hemoglobin content by percentage, as in the case of known methods of measuring hemoglobins.

The method of measuring hemoglobins of the present invention can measure the hemoglobin A1c serving as a diabetes marker. The method of measuring hemoglobin A1c by liquid chromatography utilizing the method of measuring hemoglobins of the present invention is also one aspect of the present invention.

The method of measuring hemoglobins of the present invention can simultaneously measure hemoglobin A1c together with other hemoglobins. The term "simultaneously" herein means that only a single measurement can provide the peaks of the hemoglobin A1c and other hemoglobins on the chromatogram and thereby to show the respective hemoglobin contents.

For example, hemoglobin F and hemoglobin A1c can be simultaneously measured by eluting hemoglobin F before hemoglobin A1c. The method of simultaneously measuring hemoglobin A1c and hemoglobin F by liquid chromatography utilizing the method of measuring hemoglobins of the present invention is also one aspect of the present invention.

Further, hemoglobin A1c and hemoglobin A2 can be simultaneously measured by eluting hemoglobin A2 after hemoglobin A1c. The method of simultaneously measuring hemoglobin A1c and hemoglobin A2 by liquid chromatography utilizing the method of measuring hemoglobins of the present invention is also one aspect of the present invention.

In addition, hemoglobin A1c and abnormal hemoglobins can be simultaneously measured by eluting abnormal hemoglobins before and after hemoglobin A1c. The method of simultaneously measuring hemoglobin A1c and abnormal hemoglobins by liquid chromatography utilizing the method of measuring hemoglobins of the present invention is also one aspect of the present invention.

### - Advantageous Effects of Invention

The present invention provides a method of measuring hemoglobins by liquid chromatography, in which a column is used, the column being filled with, as a column-packing material, cation-exchangeable particles including cross-linked polymer particles having a cation-exchange-group-containing polymer bonded to the surface of the cross-linked polymer particles, and the column showing a pressure value of 9.8 × 10³ Pa or higher and 29.4 × 10⁵ Pa or lower when an eluent for the measurement is delivered at a rate of 1.0 mL/min. Use of the column satisfying the above conditions can solve the problems in conventional measurement under high-pressure conditions and enables to measure hemoglobins in a shorter time at high accuracy. Further, the present invention enables to simultaneously measure hemoglobin A1c and other hemoglobins.

### BRIEF DESCRIPTION OF THE DRAWINGS

Fig. 1 shows one exemplary configuration of a liquid chromatograph used in the method of measuring hemoglobins of the present invention.
Fig. 2 shows a chromatogram obtained in the measurement of hemoglobin A1c in healthy human blood with the column and the measurement conditions in Example 1.
Fig. 3 shows a chromatogram obtained in the measurement of abnormal hemoglobins with the column and the measurement conditions in Example 1.
Fig. 4 shows a chromatogram obtained in the measurement of hemoglobin A2 with the column and the measurement conditions in Example 1.
Fig. 5 shows a chromatogram obtained in the measurement of hemoglobin A1c in healthy human blood with the column and the measurement conditions in Comparative Example 1.
Fig. 6 shows a chromatogram obtained in the measurement of abnormal hemoglobins with the column and the measurement conditions in Comparative Example 2.
Fig. 7 shows a chromatogram obtained in the measurement of hemoglobin A2 with the column and the measurement conditions in Comparative Example 2.
Fig. 8 illustrates graphs each showing the relationship between the pressure value in measurement and the CV value in the simultaneous repeatability test with the columns in Example 1 and Comparative Example 4.
Fig. 9 illustrates graphs each showing the relationship between the pressure value in measurement and the CV value in the simultaneous repeatability test with the columns in Example 1 and Comparative Example 2.
Fig. 10 illustrates graphs each showing the relationship between the pressure value in measurement and the CV value in the simultaneous repeatability test with the columns in Example 1 and Comparative Example 3.
Fig. 11 illustrates graphs showing changes in hemoglobin A1c value of Measurement Examples 1 to 7 in which the healthy human blood was repeatedly measured.
Fig. 12 illustrates graphs showing changes in CV value in the simultaneous repeatability test of Measurement Examples 1 to 7 in which the healthy human blood was repeatedly measured.

### DESCRIPTION OF EMBODIMENTS

The present invention will be described in detail below referring to, but not limited to, examples.

In each of Examples 1 to 4, a column-packing material was prepared which includes cross-linked polymer particles having a cation-exchange-group-containing polymer bonded to the surface of the cross-linked polymer particles, and a column was also prepared which satisfies the "specified column pressure value" of the present invention. Examples 1 to 4 each provide the measurement with this column-packing material under the conditions satisfying the "specified pressure value in measurement" of the present invention.

### (Example 1)

Benzoyl peroxide (1.0 g, NACALAI TESQUE, INC.) was dissolved in tetraethylene glycol dimethacrylate (200 g, Shin Nakamura Chemical Co., Ltd.), diethylene glycol dimethacrylate (50 g, Shin Nakamura Chemical Co., Ltd.), and tetramethylolmethane triacrylate (100 g, Shin Nakamura Chemical Co., Ltd.). The mixture obtained was dispersed in a solution (2 L) of 5% by weight polyvinyl alcohol ("Gohsenol GH-20", The Nippon Synthetic Chemical Industry Co., Ltd.) in water, and the dispersion was warmed to 80°C in a nitrogen atmosphere with stirring using rotary blades at a rotation rate of 300 rpm, thereby proceeding one-hour polymerization reaction. One hour later, a solution (200 mL) of acrylamide-tert-butyl sulfonate (80 g, TOAGOSEI CO., LTD.) in water was added to the reaction system, and the polymerization reaction was continued for another one hour at 80°C. This provided cation-exchangeable particles (column-packing material) including cross-linked polymer particles having a cation-exchange-group-containing polymer bonded to the surface of the cross-linked polymer particles.

The obtained column-packing material was washed, and then the particle size of the column-packing material was measured using a particle size analyzer (Nicomp, "AccuSizer 780"). The measurement showed that the volume average particle size was 9.3 µm.

The column-packing material (0.8 g) was added to a 50 mmol/L phosphate buffer (pH: 6.0, 30 mL) and the mixture was stirred. Then, the mixture was sonicated for five minutes to provide a slurry. The whole slurry was charged into a column-packing reservoir (capacity: 30 mL, AS ONE Corp.) connected with a stainless-steel empty column (internal diameter of column body: 4.6 mm, length: 35 mm) and other components. A delivery pump ("PU-614", GL Sciences Inc.) was connected to the column-packing reservoir and the slurry was charged at a pressure of 20 MPa, thereby providing a column for measuring hemoglobins.

The obtained column (separation column 7) for measuring hemoglobins was connected to a liquid chromatograph 1 shown in Fig. 1. A manometer 4 ("digital manometer GC61", NAGANO KEIKI CO., LTD.) was connected between a delivery pump 3 and an injection valve 5, measuring the pressure value. The measurement conditions were described below. The column pressure value when the eluent A was delivered at a flow rate of 1.0 mL/min was 1300 × 10³ Pa.

### Measurement conditions

- System: LC-20A system (Shimadzu Corp.)
- Eluent
   Eluent A: 200 mmol/L phosphate buffer (pH: 5.8)
   Eluent B: 400 mmol/L phosphate buffer (pH: 7.8)
- Eluting conditions
   0.0 minutes to 1.0 minute: eluent A occupied 100%
   1.0 minute to 1.1 minutes: eluent B occupied 100%
   1.1 minutes to 2.0 minutes: eluent A occupied 100%
- Flow rate: 1.0 mL/min
- Detection wavelength: 415 nm
- Amount of sample filled: 10 µL

The hemoglobin A1c in healthy human blood was measured using the aforementioned column under the aforementioned measurement conditions.

The measurement sample was prepared by collecting a healthy human blood with sodium fluoride and hemolytically diluting the blood 200 times with a phosphate buffer (pH: 6.8) containing 0.05% Triton X-100 (Sigma-aldrich Co.).

Fig. 2 shows the chromatogram obtained by the measurement under the aforementioned measurement conditions. In Fig. 2, the peak 21 indicates hemoglobin A1c, the peak 22 indicates hemoglobin A0, and the peak 23 indicates hemoglobin F. The hemoglobin A1c and the other hemoglobins were well separated in a short time. This measurement was repeated 20 times with the same sample, in other words, a simultaneous repeatability test was performed. As shown in Table 1, the repeatability for the hemoglobin A1c value (HbA1c) and for the hemoglobin F value (HbF) were both good.

**[Table 1]**

| | Rotation rate (rpm) | Average particle size (µm) | Empty column | | Column Pressure value (×10³ Pa) | Flow rate in measurement (mL/min) | Pressure value in measurement (×10³ Pa) | Simultaneous repeatability (CV value (%)) | | | | | Δ value (%) | | |
|---|---|---|---|---|---|---|---|---|---|---|---|---|---|---|---|
| | | | Internal diameter (mm) | Length (mm) | | | | HbF | HbA1c | HbS | HbC | HbA2 | Sample L | Sample A | Sample C |
| Example 1 | 300 | 9.3 | 4.6 | 35 | 1300 | 1.0 | 1300 | 7.30 | 0.74 | 0.95 | 0.84 | 0.94 | 0.08 | 0.14 | 0.12 |
| Example 2 | 250 | 13.8 | 4.6 | 35 | 700 | 1.0 | 700 | 6.35 | 0.72 | 0.96 | 1.25 | 1.03 | 0.16 | 0.14 | 0.12 |
| Example 3 | 200 | 22.3 | 4.6 | 35 | 200 | 1.0 | 200 | 7.49 | 0.73 | 1.05 | 1.43 | 0.84 | 0.12 | 0.17 | 0.18 |
| Example 4 | 150 | 24.7 | 4.6 | 35 | 35 | 1.0 | 35 | 9.83 | 0.84 | 1.16 | 1.66 | 1.21 | 0.16 | 0.15 | 0.19 |
| Example 5 | 350 | 7.2 | 4.6 | 35 | 2200 | 1.0 | 2200 | 13.45 | 1.23 | 2.14 | 1.67 | 1.19 | 0.15 | 0.23 | 0.31 |
| Example 6 | 400 | 6.1 | 4.6 | 35 | 2880 | 1.0 | 2880 | 12.74 | 1.06 | 2.31 | 1.86 | 1.26 | 0.14 | 0.26 | 0.28 |
| Example 7 | 350 | 7.2 | 4.6 | 25 | 1740 | 1.0 | 1740 | 8.06 | 0.73 | 1.06 | 1.06 | 1.15 | 0.14 | 0.13 | 0.14 |
| Example 8 | 400 | 6.1 | 4.6 | 25 | 2430 | 0.8 | 1900 | 7.92 | 0.78 | 1.15 | 1.12 | 1.06 | 0.13 | 0.12 | 0.13 |
| Comparative Example 1 | 100 | 42.3 | 4.6 | 35 | 6.8 | 1.0 | 6.8 | 42.50 | 10.30 | - | - | - | 2.18 | 1.98 | 2.18 |
| Comparative Example 2 | 450 | 4.2 | 4.6 | 35 | 3760 | 1.0 | 3760 | 30.70 | 3.45 | 7.41 | 8.63 | 5.38 | 1.26 | 1.41 | 2.13 |
| Comparative Example 3 | 300 | 9.3 | 4.6 | 35 | 3320 | 1.0 | 3320 | 29.70 | 3.77 | 8.32 | 9.32 | 6.32 | 1.47 | 1.59 | 1.86 |
| Comparative Example 4 | 300 | 9.4 | 4.6 | 35 | 1750 | 1.0 | 1750 | 43.00 | 4.20 | - | - | - | 2.32 | 2.40 | 2.18 |

Samples containing a modified hemoglobin were artificially prepared and were measured using the aforementioned column under the aforementioned measurement conditions.

The samples containing a modified hemoglobin were the following three samples: a sample containing labile hemoglobin A1c (sample L); a sample containing acetylated hemoglobin (sample A); and a sample containing carbamylated hemoglobin (sample C), each prepared by a known method. The sample L was prepared by adding glucose to healthy human blood so as to give a concentration of 2000 mg/dL and warming the blood at 37°C for three hours. The sample A was prepared by adding acetaldehyde to healthy human blood so as to give a concentration of 50 mg/dL and warming the blood at 37°C for two hours. The sample C was prepared by adding sodium cyanate to healthy human blood so as to give a concentration of 50 mg/dL, and warming the blood at 37°C for two hours.

The performance of separating the hemoglobin A1c and the respective modified hemoglobins was evaluated by calculating the value (Δ value) obtained by subtracting the hemoglobin A1c value of the healthy human blood used as the material for preparing a modified hemoglobin from the hemoglobin A1c value of the sample containing a modified hemoglobin (sample L, sample A, or sample C). As shown in Table 1, the Δ values were each lower than 0.2%. Thus, the hemoglobin A1c was accurately measured even in the modified hemoglobin-containing samples.

With the aforementioned column, a sample containing hemoglobin S and hemoglobin C ("AFSC Hemo Control", Helena Laboratories), which are abnormal hemoglobins, was measured. Fig. 3 shows the chromatogram obtained. In Fig. 3, the peak 24 indicates the hemoglobin S and the peak 25 indicates the hemoglobin C. The abnormal hemoglobins, i.e. hemoglobin S and hemoglobin C, were well separated. As shown in Table 1, the simultaneous repeatability tests provided good repeatability for the hemoglobin S and for the hemoglobin C.

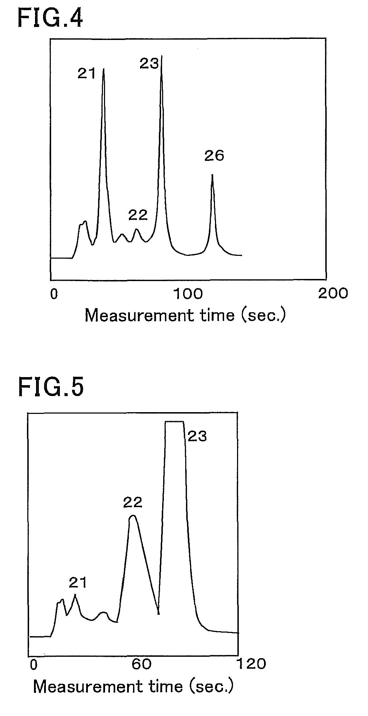

With the aforementioned column, a hemoglobin A2-containing sample ("A2 control level 2", Bio-Rad Laboratories, Inc.) was measured. Fig. 4 shows the chromatogram obtained. The peak 26 in Fig. 4 indicates the hemoglobin A2. The hemoglobin A2 was well separated. As shown in Table 1, the simultaneous repeatability test provided good repeatability for the hemoglobin A2.

### (Examples 2 to 4)

A column-packing material whose volume average particle size was different from that in Example 1 was prepared through polymerization under stirring at a smaller rotation rate than in the polymerization reaction of Example 1. The volume average particle size and the column pressure value were measured in the same manner as in Example 1. Table 1 shows the rotation rate set for the polymerization, the volume average particle size, the size of the column used, the column pressure value, the flow rate in measurement, and the pressure in measurement. Table 1 also shows the above conditions and the measurement results of the following examples and the comparative examples.

With the obtained column, the healthy human blood, the abnormal hemoglobin-containing samples, and the hemoglobin A2-containing sample used in Example 1 were measured. These measurements provided the chromatograms shown in Fig. 2, Fig. 3, and Fig. 4.

Table 1 shows the results of the simultaneous repeatability tests and the Δ values in the measurements of the modified hemoglobin-containing samples. Each value was as favorable as in the case of the column of Example 1.

### (Examples 5 and 6)

In Examples 5 and 6, a column was prepared so as to include cross-linked polymer particles having a cation-exchange-group-containing polymer bonded to the surface of the cross-linked polymer particles and adapt to the "specified column pressure value" of the present invention. The measurement of hemoglobins was performed under the conditions which do not adapt to the "specified pressure value in measurement" of the present invention.

Cation-exchangeable particles whose volume average particle size was different from that in Example 1 was prepared through polymerization under stirring with a greater rotation rate than in the polymerization reaction of Example 1. The volume average particle size and the column pressure value were measured in the same manner as in Example 1. Table 1 shows the rotation rate set for the polymerization, the volume average particle size, and the column pressure value.

With the obtained column, the healthy human blood, the abnormal hemoglobin-containing samples, and the hemoglobin A2-containing sample used in Example 1 were measured. These measurements provided the chromatograms shown in Fig. 2, Fig. 3, and Fig. 4.

Table 1 shows the results of the simultaneous repeatability tests and the Δ values of the measurements of the modified hemoglobin-containing samples. The values were each at a good level without any problem in practice, but the CV values and the Δ values were slightly greater than those in the case of the columns in Examples 1 to 4.

### (Example 7)

In Example 7, the measurements were performed using the column-packing material of Example 5 under the conditions adapting to the "specified pressure value in measurement" of the present invention.

The column-packing material of Example 5 was charged into a stainless-steel empty column (column body internal diameter: 4.6 mm, length: 25 mm) shorter than the column of Example 1. Table 1 shows the measurement result of the column pressure value. The column pressure value adapted to the "specified column pressure value" and the "specified pressure value in measurement" of the present invention.

Table 1 also shows the results of the simultaneous repeatability tests and the Δ values of the measurements of the modified hemoglobin-containing samples. The values were better than those in Example 5, and were as good as those in Examples 1 to 4.

### (Example 8)

In Example 8, the measurements were performed using the column-packing material of Example 6 under the conditions adapting to the "specified pressure value in measurement" of the present invention.

The column-packing material of Example 6 was charged into a stainless-steel empty column (column body internal diameter: 4.6 mm, length: 25 mm) shorter than the column used in Example 1. Table 1 shows the measurement result of the column pressure value. The column pressure value adapted to the "specified column pressure value" of the present invention. Additional measurement was performed under the measurement conditions in Example 1 except that the flow rate of the eluent was 0.8 mL/min instead of 1.0 mL/min. Table 1 shows the measurement result of the pressure value in measurement. The pressure value in measurement adapted to the "specified pressure value in measurement" of the present invention.

Table 1 shows the results of the simultaneous repeatability tests and the Δ values in the measurements of the modified hemoglobin-containing samples. The values were improved and were as good as the results in Examples 1 to 4.

Examples 7 and 8 confirm that the use of a column-packing material adapting to the "specified column pressure value" of the present invention provides good repeatability, and the measurement under the conditions adapting to the "specified pressure value in measurement" of the present invention results in better repeatability.

### (Comparative Example 1)

In Comparative Example 1, a column that does not adapt to the "specified column pressure value" of the present invention was prepared through polymerization under stirring at a smaller rotation rate than in the polymerization reaction of Example 1, and the hemoglobins were measured with this column.

The volume average particle size and the column pressure value were measured in the same manner as in Example 1. Table 1 shows the rotation rate set for the polymerization, the volume average particle size, and the column pressure value.

With the obtained column, the healthy human blood, the abnormal hemoglobin-containing samples, and the hemoglobin A2-containing sample used in Example 1 were measured. Fig. 5 shows the chromatogram obtained by the measurement of the healthy human blood. In comparison between Fig. 2 and Fig. 5, the column of Comparative Example 1 had poorer performance of separating the hemoglobin A1c and the other hemoglobins. Further, the column failed to separate the abnormal hemoglobins and the hemoglobin A2.

Table 1 shows the results of the simultaneous repeatability tests and the Δ values in the measurements of the modified hemoglobin-containing samples. The values were poorer than in the case of the column of Example 1.

### (Comparative Example 2)

In Comparative Example 2, a column that does not adapt to the "specified column pressure value" of the present invention was prepared through polymerization under stirring at a greater rotation rate than in the polymerization reaction of Example 1, and the hemoglobins were measured with this column.

With the obtained column, the volume average particle size and the column pressure value were measured in the same manner as in Example 1. Table 1 shows the rotation rate set for the polymerization, the volume average particle size, and the column pressure value.

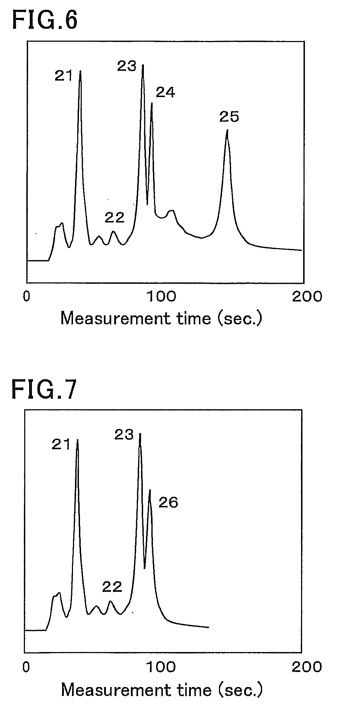

With the obtained column, the healthy human blood used in Example 1 was measured and the measurement provided a chromatogram similar to that shown in Fig. 5. The abnormal hemoglobin-containing samples and the hemoglobin A2-containing sample used in Example 1 were also measured. Fig. 6 and Fig. 7 show the chromatograms obtained. The column of Comparative Example 2 had poorer performance of separating the hemoglobin A1c and the other hemoglobins.

Table 1 shows the results of the simultaneous repeatability tests and the Δ values in the measurements of the modified hemoglobin-containing samples. The values were poorer than in the case of the column of Example 1.

### (Comparative Example 3)

In Comparative Example 3, a column that does not adapt to the "specified column pressure value" of the present invention was prepared under the conditions different from those in Example 1, except the rotation rate upon stirring in the polymerization reaction, and the hemoglobins were measured with this column.

A column-packing material was prepared in the same manner as in Example 1, except that the amount of the acrylamide-tert-butyl sulfonate used in polymerization was increased from 80 g to 160 g. The volume average particle size and the column pressure value were measured in the same manner as in Example 1. Table 1 shows the rotation rate set for the polymerization, the volume average particle size, and the column pressure value.

The chromatograms obtained in the measurements of healthy human blood, the abnormal hemoglobin-containing samples, and the hemoglobin A2-containing sample used in Example 1 were similar to those shown in Fig. 5, Fig. 6, and Fig. 7, respectively. The performance of separating the hemoglobin A1c and the other hemoglobins was poor.

Table 1 shows the results of the simultaneous repeatability tests and the Δ values in the measurements of modified hemoglobin-containing samples. The values were poorer than in the case of the column of Example 1.

### (Comparative Example 4)

In Comparative Example 4, a column was prepared so as to include no cation-exchange-group-containing polymer bonded to the surface of the cross-linked polymer particles but to adapt to the "specified column pressure value" of the present invention, and the measurement was performed under the condition adapting to the "specified pressure value in measurement" of the present invention.

Benzoyl peroxide (1.0 g, NACALAI TESQUE, INC.) was dissolved in triethylene glycol dimethacrylate (200 g), diethylene glycol dimethacrylate (50 g), tetramethylolmethane triacrylate (100 g), and acrylamide-tert-butyl sulfonate (80 g, TOAGOSEI CO., LTD.). The mixture obtained was dispersed in a solution (2 L) of 5% by weight polyvinyl alcohol ("Gohsenol GH-20", The Nippon Synthetic Chemical Industry Co., Ltd.) in water. The dispersion was warmed to 80°C in nitrogen atmosphere under stirring at a rotation rate of 300 rpm, thereby proceeding polymerization reaction for 10 hours. This yielded cation-exchangeable particles (column-packing material) including no cation-exchange-group-containing polymer bonded to the surface of the cross-linked polymer particles.

The volume average particle size and the column pressure value were measured in the same manner as in Example 1. Table 1 shows the results.

The healthy human blood, the abnormal hemoglobin-containing samples, and the hemoglobin A2-containing sample used in Example 1 were measured. The chromatogram obtained in the measurement of the healthy human blood was similar to that shown in Fig. 5. The performance of separating the hemoglobin A1c and the other hemoglobins was poor. The abnormal hemoglobins and the hemoglobin A2 were not separated at all.

Table 1 shows the results of the simultaneous repeatability tests and the Δ values in the measurements of the modified hemoglobin-containing samples. The values were poorer than in the case of the column of Example 1.

### (Evaluations)

The performance was evaluated using the column-packing material or the column in the examples and the comparative examples.

### (1) Influence of structure of column-packing material on repeatability

The evaluation (1) examined the influence of the structure of a column-packing material on the CV values in the simultaneous repeatability test performed in Example 1 using the column-packing materials of Examples 1 and Comparative Example 4 at various pressure values in measurement. The pressure values in measurement were changed as follows: the column-packing materials were charged into two empty columns with a column body length of 35 mm and 15 mm, and the flow rate was adjusted in the range from 0.5 mL/min to 2.5 mL/min.

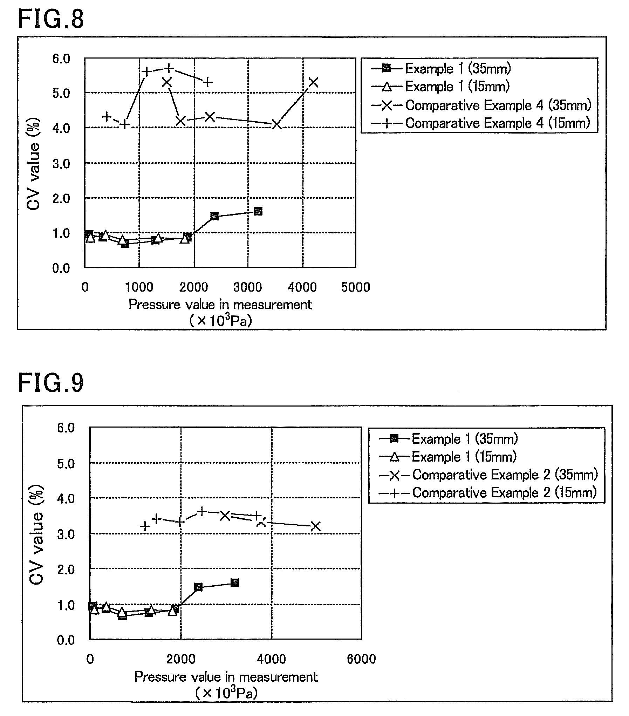

Fig. 8 shows the relationship between the pressure value in measurement and the CV value. With the column-packing material (the column-packing material obtained in Example 1) including cross-linked polymer particles having a cation-exchange-group-containing polymer bonded to the surface of the cross-linked polymer particles and the column adapting to the specified column pressure value, the repeatability was good. The repeatability was better under the conditions adapting to the specified pressure value in measurement.

With the column-packing material (the column-packing material obtained in Comparative Example 4) including no cation-exchange-group-containing polymer bonded to the surface of the cross-linked polymer particles, the repeatability was poor even under the conditions adapting to the specified column pressure value. The specified pressure value in measurement had no influence.

### (2) Influence 1 of adaptability or inadaptability to specified column pressure value on repeatability

The evaluation (2) examined the relationship between the pressure value in measurement and the CV value in the simultaneous repeatability test performed in Example 1 using the column-packing material of Comparative Example 2 in the same manner as in the evaluation (1).

Fig. 9 shows the obtained relationship between the pressure value in measurement and the CV value. The column-packing material of Comparative Example 2 was the column-packing material including cross-linked polymer particles having a cation-exchange-group-containing polymer bonded to the surface of the cross-linked polymer particles but inadaptable to the "specified column pressure value" of the present invention. In this case, the repeatability was poor and was not improved even under the conditions adapting to the specified pressure value in measurement.

### (3) Influence 2 of adaptability or inadaptability to specified column pressure value on repeatability

The evaluation (3) examined the relationship between the pressure value in measurement and the CV value in the simultaneous repeatability test performed in Example 1 using the column-packing material of Comparative Example 3 in the same manner as in the evaluation (1).

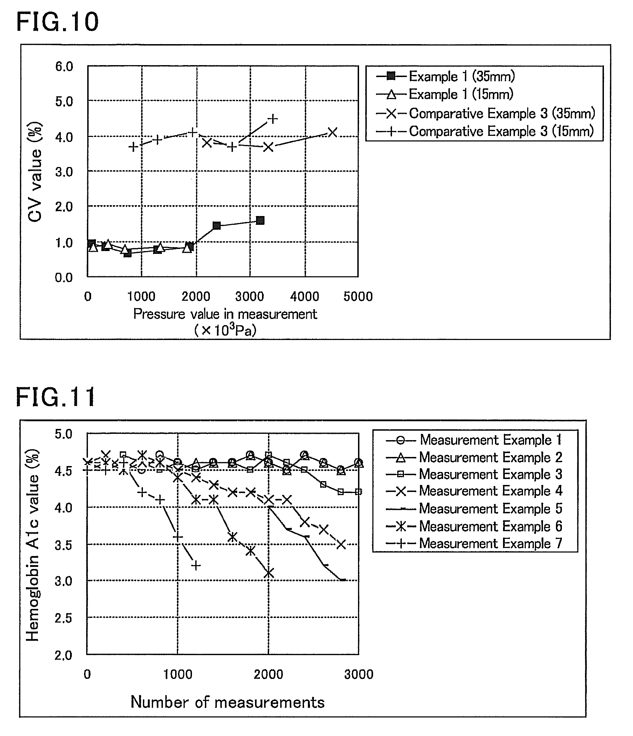

Fig. 10 shows the obtained relationship between the pressure value in measurement and the CV value. The column-packing material of Comparative Example 3 was cation-exchangeable particles including cross-linked polymer particles having a cation-exchange-group-containing polymer bonded to the surface of the cross-linked polymer particles but inadaptable to the "specified column pressure value" of the present invention. In this case, the repeatability was poor and was not improved even under the conditions adapting to the specified pressure value in measurement.

The evaluations (1) to (3) proved that achievement of good repeatability requires the use of the column-packing material including cross-linked polymer particles having a cation-exchange-group-containing polymer bonded to the surface of the cross-linked polymer particles and the use of the column adapting to the specified column pressure value of the present invention. Further, the specified pressure value in measurement provides better repeatability.

### (4) Influence of repetition of measurement

The same healthy human blood sample was repeatedly measured many times using the column-packing materials of Example 1, Example 5, Comparative Example 2, Comparative Examples 3, and Comparative Example 4, thereby examining the change in hemoglobin A1c value. At every 200 measurements, the simultaneous repeatability test in Example 1 was performed and the change in CV value was examined.

With the respective column-packing materials, columns of Measurement Examples 1 to 7 were prepared. Table 2 shows the conditions of the respective measurement examples. In Table 2, the item "Adaptability to conditions of present invention" shows "○" indicating that the conditions adapt to the above conditions or "×" indicating that the conditions do not adapt to the above conditions. For example, in the case of Measurement Example 1, the column-packing material includes cross-linked polymer particles having a cation-exchange-group-containing polymer bonded to the surface of the cross-linked polymer particles. Thus, the structure of the column-packing material adapts to the condition (indicated as ○). The pressure value when an eluent was delivered at a rate of 1.0 mL/min was 1300 × 10³ Pa. Thus, this value adapts to the specified column pressure value (indicated as ○). Further, the pressure value in measurement was 1300 × 10³ Pa. Thus, this value adapts to the specified pressure value in measurement (indicated as ○).

**[Table 2]**

| | Column- packing material | Empty column | | Column Pressure value (×10³ Pa) | Flow rate in measurement (mL/min) | Pressure value in measurement (×10³ Pa) | Adaptability to conditions of present invention | | |
|---|---|---|---|---|---|---|---|---|---|
| | | Internal diameter (mm) | Length (mm) | | | | Structure of column-packing material | Specified column pressure value | Specified pressure value in measurement |
| Measurement Example 1 | Example 1 | 4.6 | 35 | 1300 | 1 | 1300 | ○ | ○ | ○ |
| Measurement Example 2 | Example 5 | 4.6 | 25 | 1740 | 1 | 1740 | ○ | ○ | ○ |
| Measurement Example 3 | Examples 5 | 4.6 | 35 | 2200 | 1 | 2200 | ○ | ○ | × |
| Measurement Example 4 | Example 1 | 4.6 | 70 | 3140 | 1 | 3140 | ○ | × | × |
| Measurement Example 5 | Comparative Example 2 | 4.6 | 35 | 3760 | 1 | 3760 | ○ | × | × |
| Measurement Example 6 | Comparative Example 3 | 4.6 | 35 | 3320 | 0.5 | 1860 | ○ | × | ○ |
| Measurement Example 7 | Comparative Example 4 | 4.6 | 35 | 1750 | 1 | 1750 | × | ○ | ○ |

Fig. 11 shows changes in hemoglobin A1c values in the repeated measurements in these measurement examples. The hemoglobin A1c values in Measurement Example 1 and Measurement Example 2 were stable even after the measurement was repeated 3000 times. The value in Measurement Example 3 slightly decreased after the measurement was repeated 2500 times. The CV values in the simultaneous repeatability tests were stable when the measurement was repeated 3000 times.

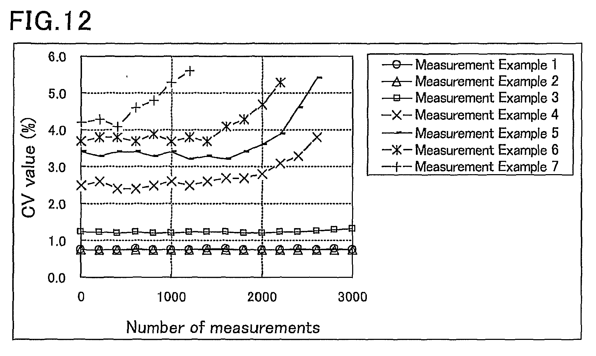

In Measurement Examples 4 to 7, the values greatly decreased before the measurement was repeated 1500 times. The changes in CV values in the simultaneous repeatability tests shown in Fig. 12 show similar tendency. The column of the present invention was proved to provide a good CV value immediately after the start of the measurement and to maintain such accuracy over many times of measurements.

### INDUSTRIAL APPLICABILITY

The present invention can provide a method of measuring hemoglobins in a short time at high accuracy. The present invention can also provide a method of measuring hemoglobin A1c, a method of simultaneously measuring hemoglobin A1c and hemoglobin F, a method of simultaneously measuring hemoglobin A1c and hemoglobin A2, and a method of simultaneously measuring hemoglobin A1c and abnormal hemoglobins.

### REFERENCE SIGNS LIST

1 : liquid chromatograph
2 : eluent-switching valve
3 : delivery pump
4 : manometer
5 : injection valve
6 : prefilter
7 : separation column
8 : detector
9 : eluent
10 : autosampler
11: waste-fluid container
21 : hemoglobin A1c
22: hemoglobin A0
23: hemoglobin F (fetal Hb)
24 : hemoglobin S
25: hemoglobin C
26: hemoglobin A2

## Claims

1. A method of measuring hemoglobins by liquid chromatography, in which a column is used,
the column being filled with, as a column-packing material, cation-exchangeable particles comprising cross-linked polymer particles having a cation-exchange-group-containing polymer bonded to the surface of the cross-linked polymer particles, and
the column showing a pressure value of 9.8 × 10³ Pa or higher and 29.4 × 10⁵ Pa or lower when an eluent for measurement is delivered at 1.0 mL/min.

2. The method of measuring hemoglobins according to claim 1,
wherein the cation-exchange-group-containing polymer is obtained by polymerizing a cation-exchange-group-containing monomer on the surface of the cross-linked polymer particles.

3. The method of measuring hemoglobins according to claim 1,
wherein the cation-exchange-group-containing polymer is obtained by polymerizing a monomer having a functional group that may be transformed into a cation exchange group on the surface of the cross-linked polymer particles.

4. The method of measuring hemoglobins according to claim 1, 2, or 3,
wherein the pressure value generated in the measurement system is set to 9.8 × 10³ Pa or higher and 19.6 × 10⁵ Pa or lower.

5. A method of measuring hemoglobin A1c by liquid chromatography, comprising:
the method of measuring hemoglobins according to claim 1, 2, 3, or 4.

6. A method of simultaneously measuring hemoglobin A1c and hemoglobin F by liquid chromatography, comprising:
the method of measuring hemoglobins according to claim 1, 2, 3, or 4.

7. A method of simultaneously measuring hemoglobin A1c and hemoglobin A2 by liquid chromatography, comprising:
the method of measuring hemoglobins according to claim 1, 2, 3, or 4.

8. A method of simultaneously measuring hemoglobin A1c and abnormal hemoglobins by liquid chromatography, comprising:
the method of measuring hemoglobins according to claim 1, 2, 3, or 4.
